# EUROPÄISCHE PATENTSCHRIFT

(11) **EP 4 441 239 B1**
(45) Veröffentlichungstag und Bekanntmachung des Hinweises auf die Patenterteilung: **23.04.2025**
(21) Anmeldenummer: 21819862.0
(22) Anmeldetag: 29.11.2021
(51) Int. Cl.: C12P 13/04, C12P 11/00, C12N 9/88

(54) **VERFAHREN ZUR HERSTELLUNG VON L-CYSTEINSÄURE**
METHOD FOR PREPARING L-CYSTEIC ACID
PROCÉDÉ DE PRÉPARATION D'ACIDE L-CYSTÉIQUE

(43) Veröffentlichungstag der Anmeldung: 09.10.2024
(73) Patentinhaber: Wacker Chemie AG, 81671 München (DE)
(72) Erfinder: PFALLER, Rupert, 80993 München (DE); SCHLÖSSER, Thomas, 84489 Burghausen (DE); WICH, Günter, 81377 München (DE)
(74) Vertreter: Belz, Ferdinand
(86) Internationale Anmeldenummer: PCT/EP2021/083372
(87) Internationale Veröffentlichungsnummer: WO 2023/094011

(56) Entgegenhaltungen:
- EP-A1- 1 247 869
- WO-A1-2023/094010
- DE-A1- 10 107 002
- MAIER T. H. P.: "Semisynthetic production of unnatural L-[alpha]-amino acids by metabolic engineering of the cysteine-biosynthetic pathway", NATURE BIOTECHNOLOGY, vol. 21, no. 4, 17 March 2003 (2003-03-17), New York, pages 422 - 427, XP093050394, ISSN: 1087-0156, DOI: 10.1038/nbt807
- ONO, K. ET AL.: "Synthesis of L-cysteine derivatives containing stable sulfur isotopes and application of this synthesis to reactive sulfur metabolome", FREE RADICAL BIOLOGY & MEDICINE, vol. 106, 9 February 2017 (2017-02-09), pages 69 - 79, XP029960555, DOI: 10.1016/J.FREERADBIOMED.2017.02.023

## Beschreibung

Die Erfindung betrifft ein Verfahren zur Herstellung von L-Cysteinsäure, wobei O-Acetyl-L-Serin (OAS) mit mindestens einem Enzym ausgewählt aus der Klasse der O-Acetyl-L-Serin-Sulfhydrylasen (OAS-Sulfhydrylasen, EC 4.2.99.8) in Gegenwart eines Salzes der schwefligen Säure umgesetzt wird, wobei es sich bei der OAS-Sulfhydrylase um CysM handelt und die Biotransformation unter aktiver pH-Kontrolle durchgeführt wird und die OAS-Konzentration im Ansatz mindestens 10 g/L beträgt. Durch diese Biotransformation wird L-Cysteinsäure zur Verfügung gestellt. Mögliche Verwendung findet L-Cysteinsäure z.B. in der Fischzucht (Nakamura et al., Fisheries Science (2021) 87: 353-363) oder im Kosmetikbereich (US4053630), z.B. als Inhaltsstoff von Regu^{®}-Slim (DSM) für die Hautpflege. In der Peptidchemie findet L-Cysteinsäure Verwendung als wasserlösliche Schutzgruppe. L-Cysteinsäure kann darüber hinaus durch Decarboxylierung zu Taurin umgewandelt werden. L-Cysteinsäure ((R)-2-Amino-3-Sulfopropionsäure, 3-Sulfo-L-Alanin, CAS 498-40-8) kann chemisch hergestellt werden z.B. durch Oxidation von Cystein mit Chlor in alkoholischer Lösung (Tao et al. Amino Acids (2004) 27: 149-151), mit Brom in HCl oder Jod-HCl in DMSO oder durch oxidative Spaltung von Cystin mit Perameisensäure. Des Weiteren kann L-Cysteinsäure auch durch Oxidation von L-Cysteinsulfinsäure hergestellt werden. Die bekannten, nicht als nachhaltig anzusehenden Verfahren zur chemischen Herstellung von L-Cysteinsäure benutzen umweltgefährdende Chemikalien und besitzen beim Verbraucher geringe Akzeptanz, insbesondere bei Anwendungen im Lebensmittel-, Kosmetik- und Pharmabereich. Daher besteht Bedarf nach einem umweltschonenderen und nachhaltigeren Herstellungsverfahren, wofür sich ein biotechnologisches Verfahren anbietet.

L-Cysteinsäure ist eine nicht-proteinogene L-Aminosäure, die in der Natur als Oxidationsprodukt der proteinogenen Aminosäure L-Cystein, z.B. in Schafwolle nachgewiesen werden kann. Cysteinsäure ist auch ein Zwischenprodukt der Coenzym M (CoM, 2-Mercaptoethansulfonsäure, CAS 3375-50-6) Biosynthese methanogener Archaebakterien.

Der Stand der Technik stellt Verfahren zur Herstellung nicht-proteinogener Aminosäuren bereit, z.B. durch direkte Fermentation von im Cysteinstoffwechsel deregulierten Mikroorganismen (EP 1 191 106 B1) oder durch OAS-Sulfhydrylase-katalysierte Biotransformation von OAS (EP 1 247 869 B1). Die Verfahren beruhen darauf, dass eine OAS-Sulfhydrylase die Reaktion von OAS mit einem Nucleophil zu einer nicht-proteinogenen Aminosäure katalysiert nach der allgemeinen Formel (1):

(1) OAS + Nucleophil -> nicht proteinogene Aminosäure + Acetat

OAS dient im Cystein-Stoffwechsel z.B. von *Escherichia coli* als biosynthetischer Vorläufer von L-Cystein. Letzteres entsteht durch Substitution der Acetat-Gruppe an der beta-Position gegen einen Thiolrest. Diese als beta-Substitution bezeichnete Reaktion wird von Enzymen der Klasse der OAS-Sulfhydrylasen (EC 4.2.99.8) katalysiert. Somit ist OAS das eigentliche Substrat (auch bezeichnet als Edukt) der OAS-Sulfhydrylase Reaktion und das Nucleophil das variable Cosubstrat.

In EP 1 247 869 B1 wurde eine Vielzahl unterschiedlicher Nucleophile auf ihre Eignung als Nucleophil für die OAS-Sulfhydrylase (z.B. CysM) katalysierte Reaktion mit OAS untersucht, darunter Selenide, Selenol, Azide, Cyanide, Azole und Isoxazolinone. Außerdem wurden auch Schwefelverbindungen aus der Gruppe der Thiosulfate und Thiole der allg. Formel H-S-R, wobei der Rest R ein einwertiger substituierter oder nicht substituierter Alkyl-, Alkoxy-, Aryl- oder Heteroarylrest war, untersucht.

Hergestellt wurden nicht-proteinogene Aminosäuren wie z.B. S-Phenyl-L-Cystein, die in der Natur nicht als Bausteine für die Proteinbiosynthese verwendet werden. Keines der offenbarten Nucleophile ermöglicht die Herstellung von L-Cysteinsäure.

Joo et al. (2018), J. Agric. Food Chem. 66: 13454 - 13463, beschreiben einen *Metabolic Engineering* Ansatz zur Herstellung von Taurin in dem Bakterium *Corynebakterium glutamicum.* In dem Stamm wurden die Gene einer L-Cysteinsäuresynthase, einer Cystein Dioxygenase und einer L-Cysteinsulfinsäure Decarboxylase heterolog exprimiert, um zur Produktion von Taurin zu gelangen. In Fig. 2 von Joo et al. (2018), J. Agric. Food Chem. 66: 13454 - 13463, werden verschiedene Stoffwechselwege zu Taurin beschrieben, darunter auch ein Weg ("L-Cysteine sulfonic acid pathway"), der ausgehend von O-Phospho-L-Serin über L-Cysteinsäure zu Taurin führt und prinzipiell auch zur Herstellung von L-Cysteinsäure geeignet wäre. In der Abbildung wird jedoch auch gezeigt, dass kein bekannter Biosyntheseweg von OAS zu L-Cysteinsäure führt, sondern nur zu L-Cystein.

Tevatia et al., Algal Research (2015) 9: 21-26, beschreiben die natürliche Produktion von Taurin in Mikroalgen, wobei als Zwischenprodukt auch L-Cysteinsäure nachgewiesen wurde. Wie in Fig. 1a) von Tevatia et al., Algal Research (2015) 9: 21-26 beschrieben, führt ein Biosyntheseweg von L-Serin zu L-Cysteinsäure ("Cysteate" in Fig. 1a). Keiner der beschriebenen Biosynthesewege führt über OAS zu L-Cysteinsäure. Der in Mikroalgen nachgewiesene intrazelluläre L-Cysteinsäure Gehalt war sehr gering und begleitet von mehreren eine Aufarbeitung erschwerenden Nebenprodukten wie Methionin, Cystein, Cysteinsulfinsäure, Hypotaurin und Taurin, sodass sich die Anzucht von Mikroalgen nicht für die Produktion von L-Cysteinsäure eignet.

US 2019/0062757 A1 (KnipBio) beschreibt in einem *Metabolic Engineering* Ansatz heterologe Produktionsstämme zur Herstellung von Taurin, wobei diese Stämme auch zur Herstellung von L-Cysteinsäure geeignet sein sollen. In den Fig. 4 bis 9 und 12 von US 2019/0062757 A1 werden verschiedene Biosynthesewege zu Taurin beschrieben, die L-Cysteinsäure als Zwischenprodukt enthalten und somit prinzipiell zur Herstellung von L-Cysteinsäure geeignet wären. Keiner dieser Biosynthesewege geht von OAS aus. Es wurden weiterhin nur Ausbeuten für Hypotaurin und Taurin angegeben, die mit max. 419 ng/ml sehr gering waren. Ausbeuten für die Produktion von L-Cysteinsäure wurden nicht genannt. Es muss angenommen werden, dass für L-Cysteinsäure keine höheren Ausbeuten erzielt werden können. Dieser *Metabolic Engineering* Ansatz ist somit für die biotechnologische Herstellung von L-Cysteinsäure nicht geeignet.

Ono et al. (Free Radical Biology and Medicine 106, S. 69-79, 2017) offenbart, dass die OAS-Sulfhydrylasen CysM und CysK aus *Salmonella entericum* LT2 analog zu den Enzymen aus *E. coli* (s. Maier, Nature Biotechnology 21, S. 422-427, 2003, Tab. 1) in der Lage sind, in hoher Ausbeute von über 70% aus den Substraten OAS und Sulfid Cystein zu bilden. Ono offenbart darüber hinaus, dass CysM und CysK aus *Salmonella entericum* LT2 zudem aus OAS in Gegenwart von Na₂SO₃ unter ansonsten gleichen Reaktionsbedingungen in geringen Mengen, d.h. in einer molaren Ausbeute von unter 0,2%, das Cystein-Derivat L-Cysteinsäure (Cysteinsulfonat, Salz der Cysteinsäure) bilden können.

Der Stand der Technik offenbart somit lediglich chemische Verfahren und kein wirtschaftliches Verfahren der Biotechnologie zur Herstellung von L-Cysteinsäure, das für den technischen Einsatz geeignet wäre.

Aufgabe der vorliegenden Erfindung war es, ein biotechnologisches Verfahren zur Herstellung von L-Cysteinsäure durch Biotransformation bereitzustellen.

Gelöst wurde die Aufgabe durch ein Verfahren zur Herstellung von L-Cysteinsäure, wobei O-Acetyl-L-Serin (OAS) mit mindestens einem Enzym ausgewählt aus der Klasse der O-Acetyl-L-Serin-Sulfhydrylasen (OAS-Sulfhydrylasen, EC 4.2.99.8) in Gegenwart eines Salzes der schwefligen Säure umgesetzt wird, wobei es sich bei der OAS-Sulfhydrylase um CysM handelt und die Biotransformation unter aktiver pH-Kontrolle durchgeführt wird und die OAS-Konzentration im Ansatz mindestens 10 g/L beträgt.

Durch dieses Verfahren wird durch Biotransformation hergestellte L-Cysteinsäure zur Verfügung gestellt.

Der Vorteil des erfindungsgemäßen Verfahrens ist, dass es sich um ein nachhaltiges und technisch umsetzbares Biotransformationsverfahren zur Herstellung von L-Cysteinsäure handelt. Auf umweltgefährdende Chemikalien kann verzichtet werden. Es werden keine Rohstoffe fossiler Herkunft verbraucht und es fallen keine giftigen chemischen Abfälle und/oder Abgase an. Das Herstellverfahren der vorliegenden Erfindung ist daher umweltschonend und nachhaltig. Zudem verlangt das Verfahren weder extreme Reaktionsbedingungen noch spezielle Anlagen und ist daher leicht technisch umsetzbar. Ein weiterer Vorteil des Verfahrens ist, dass auf diesem Wege natürliche L-Cysteinsäure hergestellt werden kann, die verstärkt nachgefragt wird. Es wurde überraschend gefunden, dass sich Salze der schwefligen Säure (im Folgenden als Sulfite bzw. SO₃²⁻bezeichnet) als Nucleophil in Reaktion (1) eignen und in einer Reaktion entsprechend Gleichung (2) die Synthese von L-Cysteinsäure ermöglichen.

(2) OAS + SO₃²⁻ -> L-Cysteinsäure + Acetat

Im Rahmen der vorliegenden Erfindung werden Herstellverfahren wie folgt unterschieden:
1. Chemische Verfahren
2. Biotechnologische Verfahren:
   a) durch *metabolic engineering*
      *Metabolic Engineering* (auch *"Pathwaydesign"* genannt) ist im Gegensatz zur Biotransformation eine Methode der Biotechnologie, bei der Stoffwechselwege eines Organismus durch Optimierung, bzw. Veränderung genetischer und regulatorischer Prozesse verändert werden. Dabei können durch Ergänzung des Genoms mit Genen von Enzymen neue oder veränderte Enzyme in einen Organismus eingeführt werden, bzw. Gene endogener Enzyme verstärkt oder abgeschwächt exprimiert werden und dadurch neue Stoffwechselwege in einem Organismus etabliert oder bestehende Stoffwechselwege verstärkt oder abgeschwächt werden. Ziel des *Metabolic Engineering* ist, dass der Organismus ein Stoffwechselprodukt entweder neu oder ein zelleigenes Stoffwechselprodukt mit erhöhter Ausbeute produziert. In ein *Metabolic Engineering*-Verfahren werden keine für das Stoffwechselprodukt spezifischen Ausgangsstoffe wie ein Enzymsubstrat, wie z.B. OAS in der vorliegenden Erfindung, eingesetzt, sondern lediglich ein Nährstoffmedium, auch bezeichnet als Anzuchtmedium, welches für das Wachstum des betreffenden Organismus erforderlich ist und zusammengesetzt ist aus einer C-Quelle (z.B. Glucose), einer N-Quelle (z.B. ein Ammoniumsalz oder ein komplexes Aminosäuregemisch wie z.B. Pepton oder Hefeextrakt) und weiteren für das Wachstum erforderlichen Salzen. Solche Nährstoffmedien sind dem Fachmann aus der mikrobiologischen Praxis bekannt.
   b) durch Biotransformation
      Biotransformation ist definiert als Überführung eines oder mehrerer Edukte in ein Produkt unter enzymatischer Katalyse, wobei das Enzymsubstrat mit dem Enzym in einen Reaktionsansatz gegeben wird. Im Reaktionsansatz wird das zugesetzte Enzymsubstrat, wie in der vorliegenden Erfindung OAS, enzymatisch umgesetzt (in der vorliegenden Erfindung durch CysM, ein Enzym ausgewählt aus der Klasse der OAS-Sulfhydrylasen (EC 4.2.99.8) in Gegenwart eines Salzes der schwefligen Säure), entsprechend Gleichung (2). Das oder die Edukte können dabei aus chemischer oder biotechnologischer Herstellung stammen. Das im erfindungsgemäßen Verfahren eingesetzte OAS kann z.B. aus chemischer Synthese oder aus biotechnologischer Produktion durch Fermentation eines Produktionsstammes stammen. Das für die enzymatische Katalyse eingesetzte Enzym stammt dabei entweder aus biotechnologischer Herstellung durch Anzucht eines Produktionsstammes, z.B. durch Fermentation oder es wird biologisches Material verwendet, welches das Enzym enthält (z.B. Pflanzen, Pilze, Algen, tierische Organe). Dabei kann die Biomasse aus der Anzucht des Produktionsstammes, bzw. das biologische Material direkt verwendet werden oder das Enzym wird je nach Erfordernissen der Biotransformation daraus isoliert. Das im erfindungsgemäßen Verfahren eingesetzte CysM-Enzym stammt aus biotechnologischer Herstellung durch Fermentation eines Produktionsstammes.

Ein natürlicher Herstellungsprozess ist definiert als ein biotechnologisches Herstellungsverfahren, bei dem keine gentechnisch veränderten Organismen (GVO) oder Produkte (Edukte, Enzyme) aus der Herstellung mit GVOs verwendet werden. In der vorliegenden Erfindung liegt dann ein natürlicher Herstellungsprozess für L-Cysteinsäure vor, wenn OAS als organisches Edukt und die OAS-Sulfhydrylase CysM nicht mit GVOs und nicht chemisch hergestellt wurden. Sulfit als Cosubstrat in Gleichung (2) ist eine anorganische Verbindung und grundsätzlich das Produkt der Lösung von SO₂ in Wasser, entsprechend den Gleichungen (4) bis (8), was nicht einer (irreversiblen) chemischen Synthese, sondern der reversiblen Hydratisierung des Gases SO₂ und der pH-abhängigen Dissoziation des Hydrats H₂SO₃ entspricht.

Selbstklonierung im Sinne des § 3 Nr. 3 Satz 4 des Gentechnikgesetzes (GenTG) ist nach einer Stellungnahme (Az.: 6790-10-02 von 1991) der Zentralen Kommission für biologische Sicherheit (ZKBS) ein Verfahren, bei dem genetisch gleiche oder verschiedene Formen nur einer Spezies einschließlich seiner Viren und Plasmide als Spender- und Empfängerorganismen dienen.

Im Rahmen der vorliegenden Erfindung ist ein Reaktionsansatz definiert als eine Mischung aus Edukt (Ausgangsstoff), Enzym und ggf. weiteren Reaktanden, bei dem das Edukt in ein Produkt überführt wird.

Die Ausbeute der Reaktion im Sinne der Erfindung ist definiert als die Menge des eingesetzten Edukts, welches unter Reaktionsbedingungen zum Produkt umgewandelt wird. Die Ausbeute kann angegeben werden in absoluter Menge (g oder mmol), als Volumenausbeute (Konzentration) in absoluter, auf das Volumen bezogene Menge Produkt (mM oder g/L) oder als relative Ausbeute von Produkt in Prozent des eingesetzten Edukts (unter Berücksichtigung der Molekulargewichte des Edukts und des Produkts), auch bezeichnet als prozentuale Ausbeute.

Fermentation ist ein Verfahrensschritt zur Herstellung (Kultivierung) von Zellkulturen im technischen Maßstab, bei dem ein bevorzugt mikrobieller Produktionsstamm unter definierten Bedingungen von Kulturmedium, Temperatur, pH, Sauerstoffzufuhr und Mediumdurchmischung zum Wachstum gebracht wird. Ziel der Fermentation ist, abhängig von der Konfiguration (genetischen Ausstattung) des Produktionsstammes, die Produktion eines Proteins/Enzyms oder eines Stoffwechselprodukts, jeweils mit möglichst hoher Ausbeute für die weitere Verwendung. Die Komponenten des erfindungsgemäßen Verfahrens, OAS und OAS-Sulfhydrylase CysM können durch Fermentation hergestellt werden. Endprodukt der Fermentation ist eine Fermenterbrühe, bestehend aus der Biomasse der Zellen des Produktionsstammes (Fermenterzellen) und dem von der Biomasse befreiten Fermentationsmedium (Fermentationsüberstand), das sich im Verlauf der Fermentation aus dem Anzuchtmedium und den von den Fermenterzellen sekretierten Stoffwechselprodukten gebildet hat. Die Zielprodukte der Fermentation können sich in den Fermenterzellen oder im Fermentationsmedium befinden. So findet sich OAS im Fermentationsmedium wieder, während das Enzym OAS-Sulfhydrylase sich in den Fermenterzellen wiederfindet.

Als offener Leserahmen (open reading frame, ORF, gleichbedeutend mit cds, coding sequence) wird derjenige Bereich der DNS bzw. RNS bezeichnet, der mit einem Startcodon beginnt und mit einem Stopcodon endet und für die Aminosäuresequenz eines Proteins codiert. Der ORF wird auch als codierende Region oder Strukturgen bezeichnet.

Als Gen wird der DNS-Abschnitt bezeichnet, der alle Grundinformationen zur Herstellung einer biologisch aktiven RNS enthält. Ein Gen enthält den DNS-Abschnitt, von dem durch Transkription eine einzelsträngige RNS-Kopie hergestellt wird und die Expressionssignale, die an der Regulation dieses Kopiervorgangs beteiligt sind. Zu den Expressionssignalen zählen z.B. mindestens ein Promotor, ein Transkriptions-, ein Translationsstart und eine Ribosomenbindestelle (RBS). Des Weiteren sind als Expressionssignale ein Terminator und ein oder mehrere Operatoren möglich.

Als Genkonstrukt wird ein DNS-Molekül bezeichnet, bei dem ein Gen verknüpft ist mit weiteren genetischen Elementen (z.B. Promotor, Terminator, Selektionsmarker, Replikationsursprung). Ein Genkonstrukt im Rahmen der Erfindung ist ein zirkuläres DNS-Molekül und wird als Plasmid, Vektor, bzw. Expressionsvektor bezeichnet. Die genetischen Elemente des Genkonstrukts bewirken seine extrachromosomale Vererbung während des Zellwachstums sowie die Produktion des vom Gen codierten Proteins.

L-Cysteinsäure aus der erfindungsgemäßen Biotransformation von OAS mit einem Salz der schwefligen Säure kann entweder ohne weitere Aufarbeitungsschritte direkt weiterverwendet oder aber mittels bekannter Methoden angereichert oder gereinigt werden. Dabei ist der Grad der Anreicherung abhängig von der weiteren Verwendung. Solche Methoden sind dem Fachmann aus Verfahren zur Isolierung von Aminosäuren bekannt. Sie umfassen z.B. Filtration, Zentrifugation, Extraktion, Adsorption, Ionenaustauscher-Chromatographie, Präzipitation, Kristallisation.

In einer bevorzugten Ausführungsform ist das Verfahren dadurch gekennzeichnet, dass L-Cysteinsäure aus dem Reaktionsansatz angereichert wird. Besonders bevorzugt ist dabei die Abtrennung der partikulären Biomasse, z.B. durch Zentrifugation.

In einer weiteren bevorzugten Ausführung ist das Verfahren dadurch gekennzeichnet, dass die im erfindungsgemäßen Verfahren hergestellte L-Cysteinsäure direkt weiterverwendet wird, d.h. der L-Cysteinsäure enthaltende Reaktionsansatz wird ohne weitere Aufarbeitungs-, Reinigungs- oder Isolierungsschritte wie u.a. Filtration, Zentrifugation, Extraktion, Adsorption, Ionenaustauscher-Chromatographie, Präzipitation, Kristallisation weiterverwendet. OAS-Sulfhydrylasen sind bisher aus verschiedensten Pflanzen und Mikroorganismen isoliert worden. In *E. coli* z.B. existieren zwei OAS-Sulfhydrylase-Enzyme, die als CysK und CysM bezeichnet werden. Die zugehörigen Gene sind ebenfalls bekannt und tragen die Bezeichnung cysK bzw. cysM. CysM OAS-Sulfhydrylasen im Sinne der vorliegenden Erfindung sind dadurch gekennzeichnet, dass sie die Synthese der proteinogenen Aminosäure L-Cystein aus OAS nach Gleichung (3) katalysieren können, wobei in diesem Fall Sulfid als Nucleophil dient.

(3) OAS + S²⁻ -> L-Cystein + Acetat

Obwohl beide Enzyme einen sehr ähnlichen Reaktionsmechanismus besitzen und an der Biosynthese von L-Cystein beteiligt sind, besitzt CysM im Gegensatz zu CysK ein variables Substratspektrum bezüglich des Nucleophils, das nach Gleichung (1) mit OAS reagieren kann.

Von CysM ist z.B. bekannt, dass es im Gegensatz zu CysK in der Lage ist, eine Reaktion von OAS mit Thiosulfat zu S-Sulfocystein (CAS-Nummer 1637-71-4) zu katalysieren. Diese Reaktion spielt beim Wachstum der Bakterien mit Thiosulfat als einziger Schwefelquelle eine wichtige Rolle.

Weiterhin ist aus EP 1 247 869 B1 (Wacker) die Verwendung von CysM zur Herstellung nicht-proteinogener Aminosäuren bekannt.

Bevorzugt ist das Verfahren dadurch gekennzeichnet, dass es sich bei der OAS-Sulfhydrylase CysM um ein bakterielles Enzym, besonders bevorzugt um CysM des Stammes *E. coli* handelt.

Schwefelige Säure bildet eine Vielzahl, in reversiblen Gleichgewichten gleichzeitig vorliegender chemischer Spezies, deren jeweilige Eignung als Nucleophil in der erfindungsgemäßen Biotransformation nicht vorhersehbar war. So ist es bekannt, dass schwefelige Säure (H₂SO₃) die wässrige Lösung von gasförmigen SO₂ ist und als zweiwertige Säure, abhängig vom pH der wässrigen Lösung, in unterschiedlichen Gleichgewichten vorliegt, deren Spezies auch unterschiedlich als Nucleophil geeignet sind. Die folgenden Gleichgewichte (4) bis (8) sind bekannt:

(4) SO₂(gasförgmig) <-> SO₂(gelöst)

(5) SO₂(gelöst) + H₂O <-> H₂SO₃

(6) H₂SO₃ <-> HSO₃⁻ + H⁺

(7) HSO₃⁻ <-> SO₃²⁻ + H⁺

(8) 2 HSO₃⁻ <-> S₂O₅²⁻ + H₂O

Schwefelige Säure und ihre Salze werden in der Lebensmittelindustrie als Konservierungsstoffe eingesetzt, da sie antimikrobielle Wirkung entfalten. Das bedeutet, dass schwefelige Säure und ihre Salze Mikroorganismen abtöten können, was auf die Inaktivierung der für die Lebensfähigkeit des Mikroorganismus notwendigen Enzyme zurückzuführen ist. Daher würde der Fachmann erwarten, dass auch das CysM-Enzym bei Einsatz von schwefeliger Säure oder deren Salze inaktiviert wird und L-Cysteinsäure mit dem in EP 1 247 869 B1 offenbarten Verfahren nicht zugänglich ist.

Aus den genannten Gründen war es für den Fachmann überraschend, dass unter Einsatz von Sulfit und OAS in einer Biotransformation L-Cysteinsäure hergestellt werden kann. Grundsätzlich sind alle denkbaren Salze der schwefligen Säure für die Reaktion geeignet. Bevorzugt ist das Verfahren dadurch gekennzeichnet, dass als Salz einer schwefligen Säure Na₂SO₃, K₂SO₃, (NH₄)₂SO₃, NaHSO₃ (bzw. dessen Anhydrid Na₂S₂O₅) oder KHSO₃ verwendet wird. Besonders bevorzugt wird als Salz einer schwefligen Säure Na₂SO₃, NaHSO₃ (bzw. dessen Anhydrid Na₂S₂O₅) und (NH₄)₂SO₃ und insbesondere bevorzugt Na₂SO₃ und NaHSO₃ (bzw. dessen Anhydrid Na₂S₂O₅) eingesetzt.

Denkbar ist die Verwendung von gasförmigem Schwefeldioxid, das Anhydrid der schwefligen Säure, das in den Reaktionsansatz eingebracht werden kann, wo es zur schwefligen Säure H₂SO₃ hydratisiert und je nach pH in einem Gleichgewicht mit den deprotonierten Formen HSO₃⁻ und SO₃²⁻ vorliegt.

Voraussetzung für das Verfahren ist die Verfügbarkeit von OAS. Denkbar sind chemische Verfahren zur Herstellung von OAS, z.B. durch Acetylierung von L-Serin, was aufgrund der hohen L-Serin-Preise teuer ist, oder auch die Herstellung des Racemats O-Acetyl-D/L-Serin, das direkt verwendet werden kann, bzw. aus dem Racemat wird vorher OAS gewonnen, z.B. durch Racematspaltung. Bei der direkten Acetylierung kann N-Acetyl-L-Serin (NAS) als Nebenprodukt gebildet werden, z.B. durch nicht selektive Acetylierung an der Hydroxy- oder Aminogruppe von L-Serin oder die bekannte Umlagerung von OAS zu NAS bei neutralen bis alkalischen pH-Werten (Tai et al. (1995), Biochemistry 34: 12311-12322), was die Ausbeuten erniedrigt oder das vorherige Einführen einer Schutzgruppe an der Aminogruppe von L-Serin erfordert. Aus diesem Grund ist die direkte Acetylierung von L-Serin für ein wirtschaftliches Verfahren nicht praktikabel.

Bekannt ist auch die biotechnologische Herstellung von OAS, wie z.B. in EP 1 233 067 B1 offenbart. Hierbei kommen Organismen zum Einsatz, die einen deregulierten Cystein-Stoffwechsel aufweisen und deshalb einen hohen Spiegel an OAS bereitstellen. Damit sind kostengünstige Produktionssysteme zur Herstellung von OAS verfügbar.

In einer bevorzugten Ausführungsform ist das Verfahren dadurch gekennzeichnet, dass OAS aus fermentativer Herstellung stammt. Eine fermentative Herstellung kann sowohl mit Hilfe von GVOs, als auch mit Hilfe von Organismen, die nicht zu den GVOs zählen, erfolgen.

In einer besonders bevorzugten Ausführungsform ist das Verfahren dadurch gekennzeichnet, dass OAS fermentativ mit Hilfe von Mikroorganismen, die nicht zu den GVOs zählen, hergestellt wird, wobei es insbesondere bevorzugt ist, dass OAS fermentativ mit Hilfe des Stammes *E. coli* W3110/pACYCcysEX-GAPDH-ORF306 hergestellt wird. Die letztgenannte speziell bevorzugte Ausführungsform ist in Beispiel 1 offenbart.

Das Verfahren der vorliegenden Erfindung zur Herstellung von L-Cysteinsäure ist bevorzugt dadurch gekennzeichnet, dass es sich um ein natürliches Herstellverfahren handelt. Das bedeutet, dass im Verfahren nicht nur keine GVOs verwendet werden, sondern sowohl das Edukt OAS als auch das Enzym OAS-Sulfhydrylase aus natürlicher Herstellung stammen, also weder mit GVOs noch chemisch hergestellt werden.

Diese besonders bevorzugte Ausführungsform ist in den erfindungsgemäßen Beispielen offenbart, die ein natürliches Herstellungsverfahren für L-Cysteinsäure beschreiben, bei dem sowohl OAS als auch die OAS-Sulfhydrylase CysM natürlich hergestellt werden. Sowohl der OAS-produzierende Stamm *E. coli* W3110/pACYC-cysEX-GAPDH-ORF306 (Beispiel 1) als auch der CysM-Produktionsstamm *E. coli* DH5α/pFL145 (Beispiel 2) stammen aus einer Selbstklonierung und werden nicht als GVOs eingestuft. Darin, dass sowohl OAS als auch die OAS-Sulfhydrylase ohne den Einsatz von GVOs hergestellt werden können, liegt ein besonderer Vorteil der Erfindung, denn sie offenbart ein natürliches Herstellungsverfahren für L-Cysteinsäure, woran aufgrund der möglichen Anwendungen im Futtermittelbereich und in der Kosmetik mittlerweile ein großes Interesse besteht.

Der Fachmann kann mittels Isotopenanalyse feststellen, ob ein Stoff wie beispielsweise OAS, das er als Edukt in das Verfahren einsetzen will, aus chemischer oder fermentativer Herstellung stammt. Ein zur Unterscheidung geeignetes Verfahren der Isotopenanalyse ist z.B. in Sieper et al., Rapid Commun. Mass Spectrom. (2006) 20: 2521-2527 beschrieben und beruht auf der Bestimmung der Isotopenverhältnisse für z.B. C oder N, welche verschieden sind, je nachdem ob ein Produkt aus chemischer (Erdöl-basierter) oder fermentativer (aus Pflanzenbasierten Rohstoffen) Herstellung stammt.

Ein Vorteil der vorliegenden Erfindung ist es, dass eine OAShaltige Fermenterbrühe wie sie z.B. aus einer nach EP 1 233 067 durchgeführten Fermentation erhalten wird, als OAS-Quelle nach Abtrennen der partikulären Biomasse wie z.B. durch Zentrifugation ohne weitere Aufarbeitungs-, Reinigungs- oder Isolierungsschritte wie u.a. Extraktion, Adsorption, Ionenaustauscher-Chromatographie, Präzipitation, Kristallisation direkt im erfindungsgemäßen Verfahren eingesetzt werden kann. Diese Vorgehensweise ist besonders ökonomisch und vermeidet die Isolierung einer instabilen Verbindung.

Ein fermentatives Verfahren zur Herstellung von OAS ist offenbart in EP 1 233 067 B1 und in Beispiel 1 der vorliegenden Erfindung beschrieben, wobei der Stamm *E. coli* W3110/pACYC-cysEX-GAPDH-ORF306 verwendet wird. Dieser Stamm ist nach Budapester Vertrag bei der DSMZ (Deutsche Sammlung für Mikroorganismen und Zellkulturen GmbH, Braunschweig) unter der Nummer DSM 13495 hinterlegt.

Das Verfahren ist bevorzugt dadurch gekennzeichnet, dass die OAS-Sulfhydrylase CysM aus fermentativer Herstellung stammt, besonders bevorzugt fermentativ mit Hilfe von Mikroorganismen, die nicht zu den GVOs zählen, hergestellt wird und insbesondere bevorzugt mit Hilfe eines *E. coli* Stamms, darunter im speziellen bevorzugt mit Hilfe des Stamms *E. coli* DH5α/pFL145 hergestellt wird.

Eine Durchführung der fermentativen biotechnologischen Herstellung von CysM mit dem Stamm *E. coli* DH5α/pFL145 ist in Beispiel 2 offenbart. Der Produktionsstamm besteht aus einem Wirtsstamm, wie in diesem Fall *E. coli* DH5α und einem Genkonstrukt, das für die Expression der OAS-Sulfhydrylase geeignet ist, bevorzugt das Genkonstrukt pFL145. Wirtsstamm und Genkonstrukt sowie die Herstellung des Produktionsstamms sind in EP 1 247 869 B1 (Wacker) beschrieben. Der Produktionsstamm ist hinterlegt nach Budapester Vertrag bei der DSMZ Deutsche Sammlung für Mikroorganismen und Zellkulturen GmbH (Brauschweig) unter der Nummer DSM 14088.

Die durch Fermentation gewonnene OAS-Sulfhydrylase CysM kann im erfindungsgemäßen Verfahren entweder als nicht weiter aufgearbeitete Fermenterbrühe eingesetzt werden oder aber auch als Zellsuspension nach Reisolierung der Zellen aus der Fermenterbrühe, z.B. durch Zentrifugation. Weiterhin kann die OAS-Sulfhydrylase in Form eines Zellhomogenats nach mechanischem Aufschluss der Zellsuspension oder in Form chemisch permeabilisierter Zellen (z.B. durch Chloroform) eingesetzt werden oder aber auch als Zellextrakt nach Abtrennung partikulärer Bestandteile aus dem Zellhomogenat oder auch als z.B. chromatographisch gereinigtes Enzym. Bevorzugt ist die Verwendung der OAS-Sulfhydrylase als nicht weiter aufgearbeitete Fermenterbrühe, als Zellsuspension nach Reisolierung der Zellen aus der Fermenterbrühe oder aber als Zellhomogenat nach mechanischem Aufschluss der Zellsuspension oder in Form chemisch permeabilisierter Zellen (z.B. durch Chloroform).

Besonders bevorzugt ist die Verwendung der OAS-Sulfhydrylase als Zellsuspension nach Reisolierung der Zellen aus der Fermenterbrühe oder als Zellhomogenat.

In einer insbesondere bevorzugten Ausführungsform ist das Verfahren dadurch gekennzeichnet, dass die aus der Fermenterbrühe isolierten und resuspendierten Zellen des Produktionsstammes als OAS-Sulfhydrylase eingesetzt werden.

In einer besonders bevorzugten Ausführungsform ist das Verfahren zur Herstellung von L-Cysteinsäure dadurch gekennzeichnet, dass sowohl die OAS-Sulfhydrylase als auch OAS durch Fermentation hergestellt werden.

OAS als Edukt des erfindungsgemäßen Biotransformationsverfahrens isomerisiert ab einem pH-Wert von ca. pH 7 zu N-Acetyl-L-Serin und ist dann nicht mehr für die Reaktion mit Sulfit zu L-Cysteinsäure geeignet. Der Mechanismus der Reaktion wurde in Tai et al. (1995), Biochemistry 34: 12311-12322 untersucht und beruht auf einem intramolekularen, nucleophilen Angriff der deprotonierten Aminogruppe am Carbonyl-Kohlenstoff des Acyl-Rests. Diese Reaktion wird mit abnehmendem pH-Wert unterdrückt, so dass die Verbindung z.B. bei pH 4, 0 stabil ist.

Das erfindungsgemäße Biotransformationsverfahren zeichnet sich somit dadurch aus, dass die Reaktion von OAS zu L-Cysteinsäure unter pH-Bedingungen durchgeführt wird, welche die Isomerisierung von OAS zu N-Acetyl-L-Serin minimieren.

Bevorzugt ist das Verfahren dadurch gekennzeichnet, dass die Reaktion bei einem pH-Wert durchgeführt wird, der mindestens 5,5 beträgt und ≤7,5, besonders bevorzugt ≤7,0 und insbesondere bevorzugt ≤6,5 ist.

In einer weiteren bevorzugten Ausführung des Biotransformationsverfahrens wird das Substrat OAS in einem sog. Zulaufverfahren in den Reaktionsansatz aus OAS-Sulfhydrylase und Sulfit zudosiert (Beispiel 5). Dabei wird im zudosierten OAS bevorzugt ein pH ≤6,5, besonders bevorzugt ein pH ≤6,0 und insbesondere bevorzugt ein pH ≤5,5 eingestellt, der die Isomerisierung zu N-Acetyl-L-Serin unterdrückt. Gleichzeitig wird der pH im Reaktionsansatz so eingestellt, dass er die Reaktion zu L-Cysteinsäure begünstigt. Entsprechend Gleichung (2) wird bei der Reaktion von OAS zu L-Cysteinsäure Essigsäure in stöchiometrischen Mengen freigesetzt, was im Verlauf der Reaktion zu einer Absenkung des pH-Werts im Ansatz führen kann. Da ein zu niedriger pH-Wert die Aktivität der OAS-Sulfhydrylase beeinträchtigt, muss ein zu starkes Absinken des pH-Werts verhindert werden. Dies kann passiv durch einen geeigneten hochkonzentrierten Puffer im Ansatz erfolgen oder erfindungsgemäß aktiv durch eine Mess- und Regeleinheit bewerkstelligt werden.

Erfindungsgemäß ist die aktive pH-Kontrolle durch eine Mess- und Regeleinheit, wie in Beispiel 5 offenbart, die bei Abweichung des pH-Wertes vom Sollwert durch Zudosierung einer Lauge oder Säure den gewünschten pH-Wert wieder einstellt (sog. pH-Stat Methode).

Die Reaktionstemperatur wird bevorzugt zwischen 5°C und 70°C gewählt. Bevorzugt ist eine Reaktionstemperatur zwischen 10°C und 60°C, besonders bevorzugt zwischen 15°C und 50°C und insbesondere bevorzugt zwischen 20°C und 40°C.

Das Verfahren zur Herstellung von L-Cysteinsäure erfolgt bevorzugt im wässrigen Milieu, d.h. als Lösemittel für die Reaktion wird bevorzugt Wasser verwendet.

Das erfindungsgemäße Verfahren zur Herstellung von L-Cysteinsäure kann in diskontinuierlicher oder kontinuierlicher Weise betrieben werden. Im diskontinuierlichen Betrieb (Batch Betrieb) werden dem Ansatz im Verlauf der Reaktion alle Reaktanden zugeführt und nach Beendigung der Reaktion der Ansatz aufgearbeitet. Im kontinuierlichen Betrieb wird während der Reaktion permanent OAS, OAS-Sulfhydrylase und ein Salz der schwefligen Säure zudosiert und gleichzeitig dem Ansatz eine Lösung enthaltend das Produkt L-Cysteinsäure entnommen. Es wird ein Fließgleichgewicht eingestellt, bei dem die Reaktanden so zudosiert werden, dass sie während der Verweilzeit im Reaktionsgefäß zum Produkt L-Cysteinsäure abreagieren können. Ein Verfahren zur kontinuierlichen Produktion unnatürlicher Aminosäuren ist z.B. in EP 1 247 869 B1 (Wacker) offenbart.

Bevorzugt ist das erfindungsgemäße Verfahren zur Herstellung von L-Cysteinsäure in diskontinuierlicher Weise.

Bevorzugt ist das Verfahren dadurch gekennzeichnet, dass die Konzentration des Salzes der schwefligen Säure mindestens in äquimolarer Konzentration, besonders bevorzugt in mindestens 1,5-fach molarem Überschuss, insbesondere bevorzugt in mindestens 2-fach molarem Überschuss und darüber hinaus bevorzugt in mindestens 5-fach molarem Überschuss zu OAS vorliegt.

Die OAS-Konzentration im Ansatz beträgt 10 g/L und bevorzugt mindestens 40 g/L.

Bei der Biotransformation von OAS beträgt die molare Ausbeute an L-Cysteinsäure bezogen auf die molare Einsatzmenge OAS bevorzugt mindestens 60%, besonders bevorzugt mindestens 70% und insbesondere bevorzugt mindestens 80%.

Die Erfindung wird durch die folgenden Beispiele weiter erläutert:

### Beispiel 1: Herstellung von OAS

Verwendet wurde der in EP 1 233 067 B1 (Wacker) offenbarte Stamm *E. coli* W3110/pACYC-cysEX-GAPDH-ORF306, hinterlegt nach Budapester Vertrag bei der DSMZ Deutsche Sammlung für Mikroorganismen und Zellkulturen GmbH (Braunschweig) unter der Nummer DSM 13495. OAS wurde wie in EP 1 233 067 B1 beschrieben durch Fermentation hergestellt. Am Ende der Fermentation wurde zur Stabilisierung von OAS ein pH-Wert von 4,5 mit 21 % (v/v) Phosphorsäure eingestellt. Die Zellen wurden durch 10 min Zentrifugation bei 4000 rpm (Heraeus Megafuge 1.0 R) abgetrennt. Der durch HPLC bestimmte Gehalt an OAS im Fermentationsüberstand betrug 15,3 g/L.

HPLC-Analytik von OAS und L-Cysteinsäure:
Zur quantitativen Bestimmung der in den Beispielen analysierten Verbindungen wurde eine jeweils für OAS und L-Cysteinsäure kalibrierte HPLC-Methode eingesetzt, wobei alle zur Kalibrierung verwendeten Referenzsubstanzen kommerziell erhältlich waren (Sigma-Aldrich). Verwendet wurde ein HPLC-Gerät der Fa. Agilent, Modell 1260 Infinity II, ausgerüstet mit einer aus der Analytik von Aminosäuren bekannten Vorsäulenderivatisierung mit o-Phtaldialdehyd (OPA-Derivatisierung) vom gleichen Hersteller. Zum Nachweis der OPA-derivatisierten Produkte OAS und L-Cysteinsäure war das HPLC-Gerät mit einem Fluoreszenzdetektor ausgerüstet. Der Detektor war eingestellt auf eine Excitationswellenlänge von 330 nm und eine Emissionswellenlänge von 450 nm. Des Weiteren verwendet wurde eine Accucore^{™} aQ Säule der Fa. Thermo Scientific^{™}, Länge 100 mM, innerer Durchmesser 4,6 mm, Partikelgröße 2,6 µm, im Säulenofen auf 40°C temperiert.

Laufmittel A: 25 mM Na-Phosphat, pH 6,0. Laufmittel B:
Methanol. Die Trennung erfolgte im Gradientenmodus: 0 - 25 min, 10% Laufmittel B auf 60% Laufmittel B, gefolgt von 2 min 60% Laufmittel B auf 100% Laufmittel B, gefolgt von weiteren 2 min auf 100% Laufmittel B, bei einer Flußrate von 0,5 ml/min. Retentionszeit von L-Cysteinsäure: 3,2 min. Retentionszeit von OAS: 17,0 min.

### Beispiel 2: Herstellung des Enzyms CysM

Verwendet wurde der in EP 1 247 869 B1 (Wacker) offenbarte Stamm *E. coli* DH5α/pFL145, hinterlegt nach Budapester Vertrag bei der DSMZ Deutsche Sammlung für Mikroorganismen und Zellkulturen GmbH (Brauschweig) unter der Nummer DSM 14088. CysM-Enzym wurde sowohl durch Anzucht im Schüttelkolben als auch durch Fermentation hergestellt.
A) Anzucht im Schüttelkolben: Vom Stamm *E. coli* DH5α/pFL145 wurde in LBamp-Medium (10 g/l Trypton (GIBCO^{™}), 5 g/l Hefeextrakt (BD Biosciences), 5 g/l NaCl, 100 mg/L Ampicillin (Sigma-Aldrich)) eine Vorkultur hergestellt (Anzucht bei 37°C und 120 rpm über Nacht). 25 ml Vorkultur wurden als Inokulum einer Hauptkultur von 250 ml LBamp-Medium (1 L Erlenmeyerkolben mit Schikane) verwendet. Die Hauptkultur wurde bei 30°C und 110 rpm geschüttelt. Nach 4 h wurde eine Zelldichte OD₆₀₀ von 1,0/ml erreicht (OD₆₀₀: photometrische Bestimmung der Zelldichte/ml Zellsuspension durch Bestimmung der Extinktion bei 600 nm; Genesys^{™} 10S UV-Vis Spektralphotometer der Fa. Thermo Scientific^{™}). Dann wurde der Induktor Tetracyclin (Sigma-Aldrich, 3 mg/L Endkonzentration) zugegeben und die Anzucht für weitere 20 h bei 30°C und 110 rpm fortgesetzt. Nach Beendigung der Anzucht betrug die Zelldichte OD₆₀₀ 3/ml.
B) Die fermentative Herstellung von CysM mit dem Stamm *E. coli* DH5α/pFL145 ist in EP 1 247 869 B1 offenbart. Die Zellen aus der Fermentation wurden durch 10 min Zentrifugation bei 4000 rpm (Heraeus Megafuge 1.0 R) abgetrennt und in KPi6,5-Puffer (0,1 M K-Phosphat, pH 6,5) suspendiert, sodass die Zelldichte OD₆₀₀ 90/ml betrug.

Die Zellen aus der Schüttelkolbenanzucht oder Fermentation wurden für die weitere Verwendung durch Zentrifugation isoliert (10 min 15000 rpm, Sorvall Zentrifuge RC5C, ausgestattet mit einem SS34 Rotor). Für die im Folgenden beschriebene weitere Verwendung zur Herstellung eines Zellhomogenats wurde das Zellpellet in KPi6,5-Puffer als Zellsuspension resuspendiert. Zur Herstellung der Zellsuspension wurde so viel KPi6,5-Puffer verwendet, dass die Zelldichte OD₆₀₀ 30/ml betrug: beispielsweise wurden 50 ml Zellen aus der Schüttelkolbenanzucht mit einer OD₆₀₀ von 3/ml zentrifugiert und in 5 ml KPi6,5-Puffer resuspendiert (10-fache Konzentrierung) oder 1 ml Zellen aus der Fermentation mit einer OD₆₀₀ von 90/ml in 3 ml KPi6,5-Puffer resuspendiert (3-fache Verdünnung).

Auf diese Weise wurden die aus der Fermenterbrühe isolierten und resuspendierten Zellen des Stammes *E. coli* DH5α/pFL145, die im Folgenden als OAS-Sulfhydrylase CysM in das erfindungsgemäße Verfahren eingesetzt wurden, erzeugt.

Zur Herstellung eines Zellhomogenats wurde der Zellhomogenisator FastPrep-24^{™} 5G der Fa. MP Biomedicals verwendet. 1 ml Zellsuspension in KPi6,5-Puffer mit einer Zelldichte OD₆₀₀ 30/ml wurde in vom Hersteller vorgefertigten 1,5 ml Röhrchen mit Glaskugeln ("Lysing Matrix B") aufgeschlossen (3 × 20 sec bei einer Schüttelfrequenz von 6000 rpm mit jeweils 30 sec Pause zwischen den Intervallen). Das erhaltene Zellhomogenat wurde direkt als OAS-Sulfhydrylase (CysM-Enzym) in das erfindungsgemäße Verfahren eingesetzt oder zur Herstellung eines Zellextrakts verwendet.

Zur Herstellung eines Zellextrakts wurde das erhaltene Zellhomogenat zentrifugiert (10 min 15000 rpm, Sorvall Zentrifuge RC5C, ausgestattet mit einem SS34 Rotor) und der Überstand mit Zellextrakt benannt und als OAS-Sulfhydrylase (CysM-Enzym) in das erfindungsgemäße Verfahren eingesetzt oder zur Bestimmung der CysM-Enzymaktivität weiterverwendet.

Der Proteingehalt des Zellextrakts wurde mit einem Qubit 3.0 Fluorometer der Fa. Thermo Fisher Scientific unter Einsatz des "Qubit^{®} Protein Assay Kits" nach Angaben des Herstellers bestimmt. Der Proteingehalt des Zellextrakts aus der Schüttelkolbenanzucht betrug 5,3 mg/ml. Der Proteingehalt des Zellextrakts aus der Fermentation betrug 4, 0 mg/ml.

Die CysM-Enzymaktivität wurde bestimmt wie in EP 1 247 869 B1 (Wacker) beschrieben. Dazu wurde OAS (Sigma-Aldrich) in Gegenwart von Na₂S und Zellextrakt aus der Anzucht des Stammes *E. coli* DH5α/pFL145 bei 37°C inkubiert. Der Testansatz (0,4 ml Endvolumen) in KPi6,5-Puffer enthielt 10 mM OAS (Zugabe aus einer 200 mM Stammlösung in 500 mM Natrium-Succinat-Puffer pH 5,5), 10 mM Natriumsulfid Na₂S und 5 µl CysM enthaltenden Zellextrakt. Das bei der CysM-Reaktion entstandene Cystein wurde mit Ninhydrin (Sigma-Aldrich) nach Gaitonde (1967), Biochem. J. 104: 627-633, bestimmt. Die CysM-Enzymaktivität im Zellextrakt aus der Anzucht des Stammes *E. coli* DH5α/pFL145 im Schüttelkolben betrug 57,1 U/ml. Da die Zellen aus der Schüttelkolbenanzucht (OD₆₀₀ von 3/ml) zur Herstellung des Zellextrakts 10-fach konzentriert worden waren, betrug die Enzymaktivität in Zellen aus der Schüttelkolbenanzucht 5,7 U/ml. Die CysM-Enzymaktivität im Zellextrakt nach Fermentation des Stammes E. coli DH5α/pFL145 betrug 58,1 U/ml. Da die Zellen aus der Fermentation (OD₆₀₀ von 90/ml) zur Herstellung des Zellextrakts auf eine OD₆₀₀ von 30/ml verdünnt worden waren, betrug die Enzymaktivität in der konzentrierten (OD₆₀₀ von 90/ml) Zellsuspension der Fermenterzellen 174,4 U/ml.

Die spezifische CysM-Enzymaktivität des Zellextrakts aus der Anzucht des Stammes *E. coli* DH5α/pFL145 im Schüttelkolben betrug 10,8 U/mg Protein. Die spezifische CysM-Enzymaktivität des Zellextrakts nach Fermentation des Stammes *E. coli* DH5α/pFL145 betrug 14,5 U/mg. Unter der Annahme, dass bei der Herstellung des Zellextrakts die CysM-Aktivität vollständig aus den Zellen freigesetzt wurde, wurde die in den Zellextrakten bestimmte CysM-Enzymaktivität in den folgenden Beispielen der in CysM-Zellsuspensionen enthaltenen Enzymaktivität gleichgesetzt.

1 U/ml CysM Enzymaktivität ist definiert als Produktion von 1 µmol Cystein/min aus OAS und Na₂S unter Testbedingungen in 1 ml Zellextrakt (Volumenaktivität). Die spezifische CysM Enzymaktivität in U/mg Protein wird erhalten durch Division der Volumenaktivität des Zellextrakts (U/ml) durch die Proteinkonzentration des Zellextrakts (mg/ml) und ist definiert als CysM Enzymaktivität in U bezogen auf 1 mg Protein im Zellextrakt.

### Beispiel 3: Herstellung von L-Cysteinsäure aus kommerziell erhältlichem OAS und Na₂SO₃ mit Hilfe von CysM hergestellt in Schüttelkolbenkultur

Zwei Ansätze wurden parallel durchgeführt:
**Ansatz 1:** In einem 100 ml Erlenmeyerkolben wurden 8,25 ml NaPi6,5-Puffer (50 mM Na-Phosphat, pH 6,5) vorgelegt und nacheinander 1 ml einer 0,2 M Lösung von Na₂SO₃ in NaPi6,5-Puffer, 0,4 ml CysM Zellextrakt aus der Schüttelkolbenanzucht (aus Beispiel 2A) mit einer Aktivität von 57,1 U/ml (2,3 U/ml Endkonzentration im Ansatz) und 350 µl einer 0,2 M Lösung von OAS x HCl (Sigma-Aldrich) in 0,5 M Na-Succinat, pH 5,5 zugegeben. Das Ansatzvolumen betrug 10 ml.
**Ansatz 2:** Der Ansatz (Vergleichsansatz ohne Na₂SO₃) hatte die gleiche Zusammensetzung wie Ansatz 1. Anstelle der Na₂SO₃-Lösung erhielt Ansatz 2 1 ml NaPi6,5-Puffer.

Beide Ansätze wurden in einem Truhenschüttler (Infors) bei 37°C und 140 rpm inkubiert. Nach 1 h und 3 h wurden jeweils 1 ml der Ansätze zum Stop der Reaktion 5 min bei 80°C inkubiert, zentrifugiert und der Überstand durch HPLC analysiert. Die mittels HPLC nachgewiesene Menge an L-Cysteinsäure ist in Tab. 1 wiedergegeben.

**Tabelle 1: Mittels HPLC nachgewiesene Menge an L-Cysteinsäure in Abhängigkeit von der Reaktionszeit, wobei kommerziell erhältliches OAS, Na₂SO₃ und ein CysM-haltiger Zellextrakt eingesetzt wurden.**

| Zeit [h] | Ansatz 1 mit Na₂SO₃ L-Cysteinsäure [mg/L] | Ansatz 2 ohne Na₂SO₃ L-Cysteinsäure [mg/L] |
|---|---|---|
| 0 | 0, 0 | 0,0 |
| 1 | 78, 0 | 0,0 |
| 3 | 95, 8 | 0,0 |

### Beispiel 4: Herstellung von L-Cysteinsäure aus OAS-haltigem Kulturüberstand der Fermentation und Na₂SO₃ mit Hilfe von CysM hergestellt in Schüttelkolbenkultur

In einem 100 ml Erlenmeyerkolben wurden 1 ml Zellkulturüberstand aus der Fermentation des Stammes *E. coli* W3110/pACYC-cysEX-GAPDH-ORF306 mit einem Gehalt an OAS von 15,3 g/L (aus Beispiel 1) vorgelegt und nacheinander 6 ml NaPi6,5-Puffer, 1 ml einer 1 M Lösung von Na₂SO₃ in NaPi6,5-Puffer und 2 ml CysM-Zellsuspension aus der Schüttelkolbenanzucht (aus Beispiel 2A, Zelldichte OD₆₀₀ 30/ml; 57,1 U/ml CysM-Enzymaktivität) zugegeben. Das Ansatzvolumen betrug 10 ml. Die CysM-Enzymaktivität im Ansatz betrug 11,4 U/ml. Der Ansatz wurde in einem Truhenschüttler (Infors) bei 37°C und 140 rpm inkubiert. Nach 2 h wurde 1 ml des Ansatzes 5 min bei 80°C inkubiert, zentrifugiert und der Überstand durch HPLC auf den Gehalt an OAS und L-Cysteinsäure analysiert. Der zeitliche Verlauf der Reaktion ist in Tab. 2 zusammengefasst.

**Tabelle 2: Mittels HPLC nachgewiesene Menge an L-Cysteinsäure und OAS, wobei ein OAS-haltiger Zellkulturüberstand, Na₂SO₃ und eine CysM-haltige Zellsuspension eingesetzt wurden.**

| Zeit [h] | | OAS [mg/L] | | L-Cysteinsäure [mg/L] | |
|---|---|---|---|---|---|
| | 0 | 1530, 0 | | 0, 0 | |
| | 2 | 0, 0 | | 1473,3 | |

### Beispiel 5: Präparative Herstellung von L-Cysteinsäure durch Biotransformation von OAS bei konstantem pH

Ein doppelwandiges 0,5 L thermostatisierbares Glasgefäß (Diehm) wurde über eine Schlauchverbindung an einen Thermostaten (Lauda) angeschlossen und auf 37°C temperiert. 50 ml CysM-haltige Zellsuspension in KPi6,5-Puffer (OD₆₀₀ 90/ml, 8720 U CysM-Enzymaktivität) aus der Fermentation des Stammes DH5α/pFL145 (aus Beispiel 2B) sowie 6,6 ml einer 400 g/L Lösung von Na₂S₂O₅ (13,9 mmol, Molekulargewicht 190, 1 g/mol) in KPi6,5-Puffer wurden vorgelegt. In gelöster Form entsprach das 27,8 mmol NaHSO3 (1,78-fach molarer Überschuss zur später zudosierten OAS-Menge von 15,6 mmol). Der Ansatz wurde mit einem Magnetrührer gerührt. Der Ansatz wurde weiterhin mit einer pH-Elektrode (Mettler Toledo) ausgestattet, welche mit einer pH-Kontrolleinheit (Titrator TitroLine alpha, Schott) verbunden war, die nach den Vorgaben des Herstellers im pH-Stat Modus betrieben wurde. Unter pH-Stat Bedingungen wurde der pH im Reaktionsgefäß während der gesamten Laufzeit der Reaktion konstant beim eingestellten pH 6,5 gehalten durch Zudosierung von 2 M NaOH aus einer mit der Kontrolleinheit verbundenen Bürette. 150 ml OAS-haltiger Zellkulturüberstand (OAS-Gehalt 15,3 g/L, 2,3 g; 15,64 mmol) aus der Fermentation des Stammes *E. coli* W3110/pACYC-cysEX-GAPDH-ORF306 (Beispiel 1) wurde aus einer Vorlage über eine Pumpe (Watson Marlow Peristaltikpumpe 101U/R) mit einer Flußrate von 0,35 ml/min in den Ansatz zudosiert.

Die Reaktionsdauer betrug 19 h. Da der Ansatz in einem offenen Reaktionsgefäß durchgeführt wurde, betrug das Ansatzvolumen infolge Verdunstung nach Beendigung der Reaktion 185 ml. 0,5 h, 3 h und 19 h nach Start der Reaktion wurde je 1 ml Aliquot des Ansatzes entnommen und der Gehalt an L-Cysteinsäure durch HPLC analysiert. Der zeitliche Verlauf der Bildung von L-Cysteinsäure ist in Tab. 3 zusammengefasst. Nach 19 h Reaktionsdauer betrug L-Cysteinsäure Gehalt im Ansatz 12970 mg/L (76,65 mM), was bei einem Ansatzvolumen von 185 ml einer absoluten molaren Ausbeute von 14,18 mmol L-Cysteinsäure entsprach. Bezogen auf die eingesetzte Menge OAS von 15,64 mmol entsprach dies einer Ausbeute von 90,1 %.

**Tabelle 3: Mittels HPLC nachgewiesene Menge an L-Cysteinsäure in Abhängigkeit von der Reaktionszeit, wobei ein OAS-haltiger Fermentationsüberstand, NaHSO₃ und eine Zellsuspension von CysM-haltigen Fermenterzellen eingesetzt wurden**

| Zeit [h] | | L-Cysteinsäure [mg/L] | | L-Cysteinsäure [mM] | |
|---|---|---|---|---|---|
| 0,5 | | 758, 0 | | 4,47 | |
| 3 | | 4244, 0 | | 25,08 | |
| 19 | | 12970, 0 | | 76, 65 | |

## Patentansprüche

1. Verfahren zur Herstellung von L-Cysteinsäure, wobei O-Acetyl-L-Serin (OAS) mit mindestens einem Enzym ausgewählt aus der Klasse der O-Acetyl-L-Serin-Sulfhydrylasen (OAS-Sulfhydrylasen, EC 4.2.99.8) in Gegenwart eines Salzes der schwefligen Säure umgesetzt wird, wobei es sich bei der OAS-Sulfhydrylase um CysM handelt und die Biotransformation unter aktiver pH-Kontrolle durchgeführt wird und die OAS-Konzentration im Ansatz mindestens 10 g/L beträgt.

2. Verfahren gemäß Anspruch 1, **dadurch gekennzeichnet, dass** es sich bei der OAS-Sulfhydrylase um ein bakterielles Enzym handelt.

3. Verfahren gemäß einem oder mehreren der Ansprüche 1 oder 2, **dadurch gekennzeichnet, dass** es sich bei der OAS-Sulfhydrylase um CysM des Stammes *E. coli* handelt.

4. Verfahren gemäß einem oder mehreren der Ansprüche 1 bis 3, **dadurch gekennzeichnet, dass** die OAS-Sulfhydrylase aus fermentativer Herstellung stammt.

5. Verfahren gemäß einem oder mehreren der Ansprüche 1 bis 4, **dadurch gekennzeichnet, dass** die OAS-Sulfhydrylase fermentativ mit Hilfe von Mikroorganismen, die nicht zu den gentechnisch veränderten Organismen (GVOs) zählen, hergestellt wird.

6. Verfahren gemäß einem oder mehreren der Ansprüche 1 bis 5, **dadurch gekennzeichnet, dass** die OAS-Sulfhydrylase fermentativ mit Hilfe des Stammes *E. coli* DH5α/pFL145 hergestellt wird.

7. Verfahren gemäß einem oder mehreren der Ansprüche 1 bis 6, **dadurch gekennzeichnet, dass** OAS aus fermentativer Herstellung stammt.

8. Verfahren gemäß einem oder mehreren der Ansprüche 1 bis 7, **dadurch gekennzeichnet, dass** OAS fermentativ mit Hilfe von Mikroorganismen, die nicht zu den GVOs zählen, hergestellt wird.

9. Verfahren gemäß einem oder mehreren der Ansprüche 1 bis 8, **dadurch gekennzeichnet, dass** OAS fermentativ mit Hilfe des Stammes *E. coli* W3110/pACYC-cysEX-GAPDH-ORF306 hergestellt wird.

10. Verfahren gemäß einem oder mehreren der Ansprüche 1 bis 9, **dadurch gekennzeichnet, dass** es sich um ein natürliches Herstellverfahren handelt, wobei ein natürlicher Herstellungsprozess dadurch definiert ist, dass im Verfahren keine GVOs verwendet werden und das Edukt OAS sowie das Enzym OAS-Sulfhydrylase aus natürlicher Herstellung stammen, also nicht mit GVOs und nicht chemisch hergestellt werden.

11. Verfahren gemäß einem oder mehreren der Ansprüche 1 bis 10, **dadurch gekennzeichnet, dass** als Salz einer schwefligen Säure Na₂SO₃, K₂SO₃, (NH₄)₂SO₃, NaHSO₃ bzw. dessen Anhydrid Na₂S₂O₅ oder KHSO₃ verwendet wird.

12. Verfahren gemäß einem oder mehreren der Ansprüche 1 bis 11, **dadurch gekennzeichnet, dass** die Konzentration des Salzes der schwefligen Säure mindestens in äquimolarer Konzentration zu OAS vorliegt.

13. Verfahren gemäß einem oder mehreren der Ansprüche 1 bis 12, **dadurch gekennzeichnet, dass** die Reaktion bei einem pH-Wert durchgeführt wird, der mindestens 5,5 beträgt und kleiner als 7,5 ist.

14. Verfahren gemäß einem oder mehreren der Ansprüche 1 bis 13, **dadurch gekennzeichnet, dass** L-Cysteinsäure aus dem Reaktionsansatz angereichert wird.

## Claims

1. Process for producing L-cysteic acid, wherein O-acetyl-L-serine (OAS) is converted using at least one enzyme selected from the class of O-acetyl-L-serine sulfhydrylases (OAS sulfhydrylases, EC 4.2.99.8) in the presence of a salt of sulfurous acid, wherein the OAS sulfhydrylase is CysM and the biotransformation is carried out under active pH control and the OAS concentration in the batch is at least 10 g/L.

2. Process according to Claim 1, **characterized in that** the OAS sulfhydrylase is a bacterial enzyme.

3. Process according to one or both of Claims 1 and 2, **characterized in that** the OAS sulfhydrylase is CysM from the strain *E. coli.*

4. Process according to one or more of Claims 1 to 3, **characterized in that** the OAS sulfhydrylase stems from fermentative production.

5. Process according to one or more of Claims 1 to 4, **characterized in that** the OAS sulfhydrylase is produced fermentatively with the aid of microorganisms that are not genetically modified organisms (GMOs).

6. Process according to one or more of Claims 1 to 5, **characterized in that** the OAS sulfhydrylase is produced fermentatively with the aid of the strain *E. coli* DH5α/pFL145.

7. Process according to one or more of Claims 1 to 6, **characterized in that** OAS stems from fermentative production.

8. Process according to one or more of Claims 1 to 7, **characterized in that** OAS is produced fermentatively with the aid of microorganisms that are not GMOs.

9. Process according to one or more of Claims 1 to 8, **characterized in that** OAS is produced fermentatively with the aid of the strain *E. coli* W3110/pACYC-cysEX-GAPDH-ORF306.

10. Process according to one or more of Claims 1 to 9, **characterized in that** the process is a natural production process, a natural production process being defined by the fact that no GMOs are used in the process and the reactant OAS and the enzyme OAS sulfhydrylase stem from natural production, i.e., are not produced using GMOs and are not produced chemically.

11. Process according to one or more of Claims 1 to 10, **characterized in that** the salt of a sulfurous acid used is Na₂SO₃, K₂SO₃, (NH₄)₂SO₃, NaHSO₃ or its anhydride Na₂S₂O₅ or KHSO₃.

12. Process according to one or more of Claims 1 to 11, **characterized in that** the concentration of the salt of sulfurous acid is at least in equimolar concentration to OAS.

13. Process according to one or more of Claims 1 to 12, **characterized in that** the reaction is carried out at a pH of at least 5.5 and of less than 7.5.

14. Process according to one or more of Claims 1 to 13, **characterized in that** L-cysteic acid is enriched from the reaction batch.

## Revendications

1. Procédé de préparation d'acide L-cystéique, de l'O-acétyl-L-sérine (OAS) étant transformée avec au moins une enzyme choisie dans la classe des O-acétyl-L-sérine-sulfhydrylases (OAS-sulfhydrylases, EC 4.2.99.8) en présence d'un sel de l'acide sulfureux, l'OAS-sulfhydrylase étant la CysM et la biotransformation étant réalisée sous régulation active du pH et la concentration en OAS dans la charge étant d'au moins 10 g/l.

2. Procédé selon la revendication 1, **caractérisé en ce que** l'OAS-sulfhydrylase est une enzyme bactérienne.

3. Procédé selon l'une ou plusieurs des revendications 1 ou 2, **caractérisé en ce que** l'OAS-sulfhydrylase est la CysM de la souche *E. coli.*

4. Procédé selon l'une ou plusieurs des revendications 1 à 3, **caractérisé en ce que** l'OAS-sulfhydrylase est issue d'une production par fermentation.

5. Procédé selon l'une ou plusieurs des revendications 1 à 4, **caractérisé en ce que** l'OAS-sulfhydrylase est produite par fermentation à l'aide de microorganismes, qui ne comptent pas parmi les organismes génétiquement modifiés (OGM).

6. Procédé selon l'une ou plusieurs des revendications 1 à 5, **caractérisé en ce que** l'OAS-sulfhydrylase est produite par fermentation à l'aide de la souche *E. coli* DH5α/pFL145.

7. Procédé selon l'une ou plusieurs des revendications 1 à 6, **caractérisé en ce que** l'OAS est issue d'une production par fermentation.

8. Procédé selon l'une ou plusieurs des revendications 1 à 7, **caractérisé en ce que** l'OAS est produite par fermentation à l'aide de microorganismes, qui ne comptent pas parmi les OGM.

9. Procédé selon l'une ou plusieurs des revendications 1 à 8, **caractérisé en ce que** l'OAS est produite par fermentation à l'aide de la souche *E. coli* W3110/pACYC-cysEX-GAPDH-ORF306.

10. Procédé selon l'une ou plusieurs des revendications 1 à 9, **caractérisé en ce qu'**il s'agit d'un procédé de production naturel, un procédé de production naturel étant défini par le fait que le procédé n'utilise pas d'OGM et que le produit de départ OAS ainsi que l'enzyme OAS-sulfhydrylase sont issus d'une production naturelle, c'est-à-dire qu'ils ne sont produits ni avec des OGM ni de manière chimique.

11. Procédé selon l'une ou plusieurs des revendications 1 à 10, **caractérisé en ce qu'**on utilise comme sel de l'acide sulfureux Na₂SO₃, K₂SO₃, (NH₄) ₂SO₃, NaHSO₃ ou son anhydride Na₂S₂O₅ ou KHSO₃.

12. Procédé selon une ou plusieurs des revendications 1 à 11, **caractérisé en ce que** la concentration en sel de l'acide sulfureux représente au moins une concentration équimolaire par rapport à l'OAS.

13. Procédé selon l'une ou plusieurs des revendications 1 à 12, **caractérisé en ce que** la réaction est réalisée à un pH qui est d'au moins 5,5 et inférieur à 7,5.

14. Procédé selon l'une ou plusieurs des revendications 1 à 13, **caractérisé en ce que** l'acide L-cystéique est enrichi à partir de la charge réactionnelle.
